# EUROPEAN PATENT SPECIFICATION

(11) **EP 1 726 298 B1**
(45) Date of publication and mention of the grant of the patent: **23.07.2008**
(21) Application number: 05017678.3
(22) Date of filing: 12.08.2005
(51) Int. Cl.: A61K 9/08, A61K 31/343

(54) **Oral liquid formulations containing citalopram**
Orale flüssige Citalopram enthaltende Formulierungen
Formulations orales liquides contenant du citalopram

(30) Priority: 05.05.2005 IT TO20050301
(43) Date of publication of application: 29.11.2006
(73) Proprietor: Errekappa Euroterapici S.p.A., 20129 Milano (IT); Medi Service S.R.L., 20041 Agrate Brianza (IT); Pharmacare S.r.l., 20149 Milano (IT)
(72) Inventor: Tavazzi, Sandra, 40134 Bologna (IT)
(74) Representative: Freyria Fava, Cristina

(56) References cited:
- EP-A1- 0 347 066
- WO-A-20/04103361
- WO-A2-20/05000216
- CH-A5- 691 477
- GUTIERREZ MARCELO M ET AL: "Pharmacokinetic comparison of oral solution and tablet formulations of citalopram: A single-dose, randomized, crossover study" CLINICAL THERAPEUTICS, vol. 22, no. 12, December 2000 (2000-12), pages 1525-1532, XP002424856 ISSN: 0149-2918

## Description

The present invention relates to new oral liquid pharmaceutical formulations containing citalopram.

Citalopram hydrochloride is a drug that inhibits re-uptake of serotonin, which is used in the treatment of depression and in anxiety disorders with panic attacks, with and without agoraphobia.

The present invention relates to new formulations containing citalopram hydrochloride, namely: 1-(3-dimethylaminopropyl)-1-(4-fluorophenyl)-1,3-dihydroisobenzofuran-5-carbonitrile hydrochloride (C₂₀H₂₁FN₂O • HCl) having the following structural 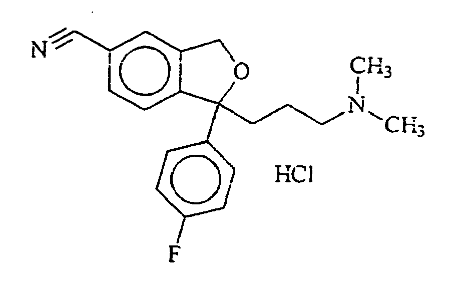 formula:

The products containing citalopram present on the market are in aqueous solution and contain preservatives and anti-microbial agents.

EP 0347066 discloses a syrup containing citalopram, sorbitol, Tragacanth, glycerol, methyl-paraben, propyl-paraben, ethanol and water.

The purpose of the present invention is to provide new oral formulations containing citalopram without the necessary presence of additives, in particular preservatives and antimicrobial agents.

According to the present invention, said purpose is achieved thanks to the solution referred to specifically in the ensuing claims. The claims form an integral part of the technical teaching provided herein in relation to the invention.

In particular, the present invention relates to new alcohol-based oral liquid formulations containing citalopram.

The invention will now be described in detail, purely by way of non-limiting example.

The new alcohol-based oral liquid formulation according to the present invention contains citalopram hydrochloride, 96% ethyl alcohol, and propylene glycol.

According to the present applicant, the use of propylene glycol as co-solvent determines an excellent chemico-physical and microbiological stability of the formulation of citalopram.

The present applicant has, in fact, found that the use of propylene glycol by itself has, in an altogether unexpected way and degree, a -much more powerful and complete action than the use of a series of compounds normally used in aqueous solutions, such as preservatives, stabilizers, densifiers and anti-microbial agents.

In what follows, formulations are described for the oral administration of citalopram hydrochloride in which the use of preservatives is excluded.

The present invention enables solutions for oral use to be obtained to be administered in the form of drops containing 40 mg per millilitre of citalopram, solubilized in a vehicle characterized by a very simple composition with a propylene glycol and 96% ethyl alcohol base.

The solution according to the present invention shows an excellent chemico-physical and microbiological stability even when exposed to light and does not require any further additives, such as for example preservatives, stabilizers and densifiers.

The solution according to the present invention enables administration, with absolute precision, of the therapeutic dose of 10, 20, 40 and 60 mg corresponding to 5, 10, 20 and 30 drops, respectively.

Ethanol in the formulation proposed performs the function of solubilizer, whilst propylene glycol is used with the principal function of co-solvent, diluent and antimicrobial agent.

The weight ratio between the two compounds, ethyl alcohol and propylene glycol, can vary in the range comprised between 0.5:9.5 and 1:1.

The following composition shows a currently preferred embodiment of the alcohol-based formulation of citalopram according to the present invention:

| | |
|---|---|
| Citalopram hydrochloride (equal to Citalopram 4.0 g) | 4.448 g |
| Ethanol 96% | 10.000 g |
| Propylene glycol | 87.252 g |
| TOT. | 101.700 g |

The present formulation is produced by means of a simplified production process, which is carried out in a short time and at a temperature lower than 30°C.

The fundamental steps of the process of production are:
(i) solubilization of citalopram in a solution of ethanol and propylene glycol in a ratio of 1:5; and
(ii) dilution to the final volume with propylene glycol.

Lots produced with the formulation described above were kept in different environmental conditions, demonstrating a perfect stability at all temperatures, such as not to require the indication of any prescription for the preservation of the product.

Of course, the details of production and the embodiments may vary widely with respect to what is described and illustrated herein, without thereby departing from the scope of the present invention, as defined by the annexed claims.

## Claims

1. An oral liquid alcohol-based pharmaceutical formulation consisting of citalopram hydrochloride, 96% ethyl alcohol, and propylene glycol.

2. The pharmaceutical formulation according to Claim 1, **characterized in that** 96% ethyl alcohol and propylene glycol are present in a weight ratio comprised in the range between 0.5:9.5 and 1:1.

3. The pharmaceutical formulation according to Claim 1 or Claim 2, **characterized in that** said formulation has a composition:
| | |
|---|---|
| Citalopram hydrochloride (equal to Citalopram 4.0 g) | 4.448 g |
| Ethanol 96% | 10.000 g |
| Propylene glycol | 87.252 g |

## Patentansprüche

1. Flüssige, alkoholbasierende pharmazeutische Oral-Formulierung, bestehend aus Citalopram-Hydrochlorid, Ethylalkohol-96% und Propylenglykol.

2. Pharmazeutische Formulierung nach Anspruch 1, **dadurch gekennzeichnet, dass** Ethylalkohol-96% und Propylenglykol in einem Gewichtverhältnis im Bereich zwischen 0,5:9,5 und 1:1 vorliegen.

3. Pharmazeutische Formulierung nach Anspruch 1 oder Anspruch 2, **dadurch gekennzeichnet, dass** die Formulierung zusammengesetzt ist aus:
| | |
|---|---|
| Citalopram-Hydrochlorid (entsprechend 4,0 g Citalopram) | 4,448 g |
| Ethanol-96% | 10,000 g |
| Propylenglykol | 87,252 g. |

## Revendications

1. Formulation pharmaceutique orale, liquide, à base d'alcool constituée de chlorhydrate de citalopram, d'alcool éthylique à 96% et de propylène glycol.

2. Formulation pharmaceutique selon la revendication 1, **caractérisée en ce que** l'alcool éthylique à 96% et le propylène glycol sont présents dans un rapport pondéral compris dans la gamme allant de 0,5 :9,5 à 1 :1.

3. Formulation pharmaceutique selon la revendication 1 ou la revendication 2, **caractérisée en ce que** la formulation présente la composition suivante :
| | |
|---|---|
| chlorhydrate de citalopram (équivalent à 4,0 g de Citalopram) | 4,448 g |
| Ethanol à 96% | 10,000 g |
| Propylène glycol | 87,252 g |
